# EUROPÄISCHE PATENTSCHRIFT

(11) **EP 4 271 453 B1**
(45) Veröffentlichungstag und Bekanntmachung des Hinweises auf die Patenterteilung: **04.12.2024**
(21) Anmeldenummer: 21847701.6
(22) Anmeldetag: 28.12.2021
(51) Int. Cl.: A61M 16/04

(54) **BEATMUNGSVORRICHTUNG MIT SPRECHLUFT-ZUFÜHRUNG**
VENTILATION DEVICE WITH VOICE AIR SUPPLY ELEMENT
DISPOSITIF DE VENTILATION AVEC ÉLÉMENT D'ALIMENTATION EN AIR VOCAL

(30) Priorität: 30.12.2020 DE 102020135139
(43) Veröffentlichungstag der Anmeldung: 08.11.2023
(73) Patentinhaber: Coloplast A/S, 3050 Humlebaek (DK)
(72) Erfinder: SCHNELL, Ralf, 63500 Seligenstadt (DE)
(86) Internationale Anmeldenummer: PCT/EP2021/087722
(87) Internationale Veröffentlichungsnummer: WO 2022/144355

(56) Entgegenhaltungen:
- EP-A1- 1 820 527
- DE-B3- 10 361 428
- DE-U1- 202004 018 665
- US-B2- 10 173 021
- US-B2- 9 038 637

## Beschreibung

Die vorliegende Erfindung betrifft eine Beatmungsvorrichtung, insbesondere eine Tracheostomiekanüle oder einen Endotrachealtubus, mit einem Kanülenrohr, einem aufblasbaren Cuff, einer Cuffluft-Leitung und einer Sprechluft-Leitung, die einen Luftauslass in dem Bereich zwischen dem Stoma und dem Cuff aufweist.

Um sicherzustellen, dass die Atemluft sowohl beim Ein- als auch beim Ausatmen nahezu vollständig durch eine Tracheostomiekanüle oder einen Endotrachealtubus strömt, ist bei vielen handelsüblichen Produkten dieser Art eine aufblasbare Manschette, der sogenannte Cuff, vorgesehen. Ein solcher Cuff dichtet die Luftröhre um die Tracheostomiekanüle bzw. um den Endotrachealtubus herum ab, damit keine Luft über die natürlichen Atemwege strömen kann. Da auf diese Weise auch keine Ausatemluft an den stimmbildenden Organen vorbeigeführt werden kann, hat ein Patient mit geblocktem Cuff typischerweise keine Möglichkeit zur Stimmerzeugung (Phonation).

Um wenigstens vorübergehend die Stimmerzeugung zu ermöglichen, kann man, sofern es die medizinische Indikation zulässt, den Cuff temporär entblocken. Wird gleichzeitig die Öffnung der Kanüle z.B. mit dem Finger oder einem Sprechventil verschlossen, kann beim Ausatmen Luft durch die natürlichen Atemwege zur Stimmerzeugung benutzt werden.

Allerdings gibt es medizinische Gründe, weshalb diese Methode bei einem erheblichen Anteil an Patienten nicht angewendet werden kann. Insbesondere bei Patienten, die einen dauerhaften Überdruck in der Lunge benötigen, hat die Beatmung bei entblocktem Cuff erhebliche Nachteile. Auch bei Patienten, die aufgrund einer Dysphagie (Schluckprobleme) einen geblockten Cuff benötigen, ist es häufig problematisch, den Cuff zum Sprechen zu entblocken, da Aspirationsgefahr besteht.

EP 1 803 478 B1 beschreibt eine Kanüle, mit deren Hilfe Patienten trotz geblocktem Cuff sprechen können. Dies wird durch eine spezielle Innenkanüle gelöst, welche die Luft beim Ausatmen mithilfe von Fenstern in der Außenkanüle so umleitet, dass die ausgeatmete Luft durch die natürlichen Atemwege fließt. Allerdings müssen hierbei bezüglich der Beatmungsparameter am Beatmungsgerät einige Kompromisse eingegangen werden, die nicht immer erwünscht sind. Bevorzugt sind daher Methoden, die keinerlei Kompromisse bei der Beatmung erfordern.

Für diese Zwecke wurde die Methode der sogenannten Above-Cuff-Vocalization (ACV) entwickelt, bei der mithilfe einer separaten Luftleitung von außen Luft in den zwischen dem Cuff und der Stimmritze liegenden subglottischen Raum eingebracht wird. Diese Luft kann dann über die natürlichen Atemwege entweichen und vom Patienten mit etwas Übung zum Sprechen verwendet werden.

Bei einigen handelsüblichen Tracheostomiekanülen ist für die Luftzuführung im subglottischen Bereich eine Sprechluft-Leitung in Form eines auf dem Kanülenrohr befestigten Schlauches vorgesehen. Das äußere Ende des Schlauches kann mit einer Druckluftquelle verbunden werden, z.B. über ein Y-Stück oder einen Konnektor. Mit einem Ventil, einem Regler oder einem Fingertip-Konnektor kann gesteuert werden, wann die Druckluft über die Sprechluft-Leitung in den subglottischen Bereich einströmt.

Anstelle von speziell für ACV hergestellten Tracheostomiekanülen werden oft Kanülen verwendet, die einen subglottischen Absaugkanal aufweisen. Der subglottische Absaugkanal dient eigentlich dazu, Sekret, das sich über dem Cuff angesammelt hat, zu entfernen. Hierfür wird an dem Absaugkanal ein Unterdruck angelegt, mit dem das Sekret aus dem subglottischen Bereich abgesaugt werden kann. Ein Beispiel für eine Tracheostomiekanüle mit subglottischer Absaugung wird in EP 1 219 317 B1 beschrieben.

Dreht man bei einer Kanüle mit subglottischem Absaugkanal die Strömungsrichtung nach dem Absaugen um, kann über den Absaugkanal Druckluft in den subglottischen Bereich eingeleitet werden, die dann als Sprechluft über die natürlichen Atemwege entweichen kann. Der Vorteil der ACV über einen subglottischen Absaugkanal besteht darin, dass durch Absaugen des Sekrets vor dem Einleiten von Sprechluft bei vielen Patienten die Sprachqualität deutlich verbessert werden kann.

Die Sprechluftzuführung über einen subglottischen Absaugkanal ist allerdings mit dem Nachteil behaftet, dass nach dem Absaugen noch an der Kanalwand befindliches Sekret durch die Druckluft eintrocknet. Das eingetrocknete Sekret klebt an der Kanalwand fest und verhärtet diese. Mit der Zeit führt dies dazu, dass der Absaugkanal undurchlässig wird und somit seine Funktion verliert. Daher ist eine separate Sprechluftzuführung erstrebenswert, die sich insbesondere auch bei solchen Kanülen bzw. Tuben gut realisieren lässt, die bereits einen subglottischen Absaugkanal aufweisen.

Neben den Vorteilen der Phonation sorgt die Belüftung der oberen Atemwege auch für einen verbesserten Abtransport von Schleim und Sekret aus den oberen Atemwegen. Zusätzlich hilft es dabei den Schluckreflex zu trainieren. Somit ist eine separate Luftzuführung in den subglottischen Bereich auch dann von Vorteil, wenn ein Patient nicht sprechen will oder kann.

Ferner sind folgende Dokumente sind aus dem Stand der Technick bekannt, nämlich US 9 038 637 B2, DE 103 61 428 B3, DE 20 2004 018665 U1, US 10 173 021 B2 und EP 1 820 527 A1.

Um die Nachteile, die mit den im Stand der Technik bereits existierenden technischen Lösungen verbunden sind, zu überwinden, wird erfindungsgemäß eine Beatmungsvorrichtung, insbesondere eine Tracheostomiekanüle oder ein Endotrachealtubus, vorgeschlagen mit
▪ einem Kanülenrohr, dessen Rohrwand einen Extrakorporalabschnitt, einen Stomaabschnitt und einen Trachealabschnitt aufweist,
▪ einem aufblasbaren Cuff der sich im Trachealabschnitt an der Außenseite der Rohrwand ringförmig um das Kanülenrohr erstreckt,
▪ einer Cuffluft-Leitung, die einen Luftauslass im Cuff aufweist,
▪ einer Sprechluft-Leitung, die einen Luftauslass in dem Bereich zwischen Stomaabschnitt und Cuff aufweist,
dadurch gekennzeichnet, dass die Sprechluft-Leitung im Bereich des Stomaabschnitts und/oder des Trachealabschnitts zumindest abschnittsweise von einer auf der Außenseite des Kanülenrohres angeordneten Folie gebildet wird, die ein variables Lumen für die Durchführung von Sprechluft ausbildet, wobei
- das variable Lumen eines von der Folie gebildeten Abschnitts n der Sprechluft-Leitung bei einem Sprechluft-Volumenstrom von 5 Litern pro Minute und einem Umgebungsdruck von 1000 mbar einen Querschnitt Qₙ aufweist,
- der Querschnitt des Lumens des Abschnitts n bei einem auf die Außenseite der Folie wirkenden Überdruck von bis zu 15 mbar um wenigstens 30% im Vergleich zum Querschnitt Qₙ reduziert wird, wenn keine Sprechluft durch die Sprechluft-Leitung strömt.

Die erfindungsgemäße Beatmungsvorrichtung kommt bei bestimmungsgemäßem Gebrauch im Bereich des Stomaabschnitts an dem Stoma eines Patienten zum Anliegen. Ausgehend von diesem Stomaabschnitt erstrecken sich der Extrakorporalabschnitt in proximaler Richtung und der Trachealabschnitt in distaler Richtung.

Die Begriffe "distal" und "proximal" werden im Sinne der vorliegenden Erfindung aus der Sicht eines die Beatmungsvorrichtung anwendenden Arztes verwendet, d.h. das proximale Ende ist das Ende der Beatmungsvorrichtung, das nach dem Einführen in die Trachea außerhalb des Patientenkörpers verbleibt, während das distale Ende in die Trachea des Patienten eingeführt wird.

Der Begriff "Leitung" wird im Zusammenhang mit der vorliegenden Erfindung im Sinne einer luftführenden Leitung verstanden, in die an dem wenigstens einen vorgesehenen Lufteinlaß Luft einströmen kann und aus der die Luft an dem wenigstens einen vorgesehenen Luftauslaß wieder ausströmen kann, nachdem die Luft durch die ansonsten luftdichte Leitung über eine bestimmte Strecke hindurchgeströmt ist.

Bei einem Sprechluft-Volumenstrom von 5 l/min, wie er typischerweise bei ACV vorliegt, nimmt die Sprechluft-Leitung in der Trachea und gegebenenfalls auch im Stoma ein bestimmtes Volumen ein. Wird der Sprechluft-Volumenstrom unterbrochen, reduziert sich dieses Volumen bereits durch geringste Druckeinwirkung, sei es durch eine Erhöhung des Umgebungsdrucks oder durch umliegendes Gewebe.

Die erfindungsgemäß vorgeschlagene Beatmungsvorrichtung ist mit dem Vorteil verbunden, dass sich das für das Hindurchleiten von genügend Sprechluft erforderliche innere Lumen dann ausbildet, wenn tatsächlich ein geeigneter Sprechluft-Volumenstrom durch die Sprechluft-Leitung strömt. Insbesondere weist die Sprechluft-Leitung bei einem Sprechluft-Volumenstrom von 5 l/min und einem Umgebungsdruck von 1000 mbar einen bestimmten Querschnitt Qₙ auf, der sich bei einem auf die Außenseite der Folie wirkenden Überdruck von bis zu 15 mbar um wenigstens 30 % verringert, sobald der Volumenstrom ausbleibt.

Erreicht wird dies dadurch, dass die Sprechluft-Leitung mittels einer zumindest abschnittsweise auf der Außenseite des Kanülenrohrs angeordneten Folie ausgebildet wird, die ein Lumen definiert, das unter den oben genannten Bedingungen in dem oben definierten Umfang variabel ist.

Der Begriff "Umgebungsdruck" bezeichnet im Sinne der vorliegenden Erfindung den auf die Folie wirkenden Druck. Bei einer nicht im Gebrauch befindlichen Beatmungsvorrichtung, die nicht in ein Stoma eingesetzt vorliegt und durch deren Sprechluft-Leitung keine Sprechluft geführt wird, entspricht der Umgebungsdruck dem Atmosphärendruck und wirkt gleichermaßen auf die nach außen und die nach innen gewandte Seite der Folie.

Wird Sprechluft durch die Sprechluft-Leitung geführt, wirkt auf die nach innen gewandte Seite der Folie ein entsprechend erhöhter Druck, der dazu führt, das die Folie in einem definierten Abschnitt n einen bestimmten Querschnitt Qₙ aufweist. Erhöht sich der von außen auf die Folie wirkende Druck, wirkt dieser erhöhte Druck dem durch die Sprechluft erhöhten Druck entgegen, der auf die nach innen gewandte Seite der Folie wirkt.

Die von außen auf die Folie wirkende Druckerhöhung kann entweder durch umliegendes Gewebe verursacht sein oder durch eine Erhöhung des von außen auf die Folie der Luftleitung wirkenden Luftdrucks.

Der Begriff "Querschnitt" bezeichnet im Zusammenhang mit der vorliegenden Erfindung die Fläche eines Querschnitts durch das Innenlumen der Sprechluft-Leitung senkrecht zur Hauptströmungsrichtung. Der Querschnitt Qₙ des Lumens der erfindungsgemäßen Sprechluft-Leitung ist gleich die Fläche des Querschnitts durch das Innenlumen in einem von der Folie gebildeten Abschnitt n der Sprechluft-Leitung bei einem Sprechluft-Volumenstrom von 5 l/min und einem Umgebungsdruck von 1000 mbar.

Typischerweise liegt der Querschnitt Qₙ des Lumens des erfindungsgemäß von der Folie gebildeten Sprechluft-Leitungsabschnitts im Bereich von 1 bis 50 mm². Bei anderen Ausführungsformen liegt der Querschnitt Qₙ im Bereich von 10 bis 40 mm². Bei bestimmten Ausführungsformen liegt der Querschnitt Qₙ des Lumens des erfindungsgemäß von der Folie gebildeten Sprechluft-Leitungsabschnitts im Bereich von 1 bis 25 mm². Bei anderen Ausführungsformen liegt der Querschnitt Qₙ im Bereich von 10 bis 25 mm².

Bei bestimmten Ausführungsformen der Erfindung reduziert sich der Querschnitt des Lumens des Abschnitts n bei einem auf die Außenseite der Folie wirkenden Überdruck von bis zu 15 mbar um höchstens 10% im Vergleich zum Querschnitt Qₙ, wenn durch die Sprechluft-Leitung Sprechluft mit einem Sprechluft-Volumenstrom von 5 Litern pro Minute strömt. Strömt keine Sprechluft mehr durch die Sprechluft-Leitung, verringert sich der Querschnitt des Lumens des Abschnitts n bei einem auf die Außenseite der Folie wirkenden Überdruck von bis zu 15 mbar um wenigstens 30%.

Vorzugsweise erstreckt sich der durch die Folie gebildete Abschnitt der Sprechluft-Leitung in der Richtung proximal-distal wenigstens über 50 %, wenigstens über 60 %, wenigstens über 70 %, wenigstens über 80 %, wenigstens über 90 % oder über 100 % der Länge des Stomaabschnitts.

Vorzugsweise erstreckt sich der durch die Folie gebildete Abschnitt der Sprechluft-Leitung im Anschluss an den Stomaabschnitt in distale Richtung über bis zu 20 %, bis zu 40 %, bis zu 60 % oder bis zu 80 % der Länge der Strecke zwischen dem Stomaabschnitt und dem Cuff.

Die Sprechluft wird typischerweise durch eine Drucklufteinrichtung bereitgestellt, die durch das Lumen der Sprechluft-Leitung einen Sprechluft-Volumenstrom von wenigstens 5 l/min bereitstellen kann. Bei dem Sprechluftvolumenstrom handelt es sich um das Volumen, das die Sprechluft-Leitung bei bestimmungsgemäßer Anwendung der erfindungsgemäßen Beatmungsvorrichtung innerhalb einer bestimmten Zeit durchströmt. Vorzugsweise handelt es sich hierbei um das Normvolumen bei standardisierter Referenzatmosphäre bei 20 °C und 1000 mbar. Vorzugsweise wird der Sprechluft-Volumenstrom bei trockener Druckluft gemessen (p(H₂O) = 0 kPA). Vorzugsweise wird das Normvolumen bei standardisierter Referenzatmosphäre gemäß DIN 1945-1 bestimmt.

Bei manchen Ausführungsformen wird Qₙ durch eine Dehnung der Folie erreicht, die sich bei einem Sprechluft-Volumenstrom von 5 l/min und einem Umgebungsdruck von 1000 mbar einstellt. Die Reduzierung von Qₙ durch erhöhten Umgebungsdruck bzw. durch verringerten Volumenstrom stellt sich bei diesen Ausführungsformen dadurch ein, dass die Dehnung der Folie aufgrund ihrer Elastizität wieder zurückgeht. Bei anderen Ausführungsformen wird Qₙ dadurch erreicht, dass bei einem Sprechluft-Volumenstrom von 5 l/min und einem Umgebungsdruck von 1000 mbar die Folie durch die Sprechluft, je nach Ausführungsform entgegen der Schwerkraftwirkung bzw. der Eigenspannung der Folie, aufgespannt wird, ohne dass hierbei ein Dehnung der Folie erfolgt. Die Reduzierung von Qₙ durch erhöhten Umgebungsdruck bzw. durch verringerten Volumenstrom stellt sich bei diesen Ausführungsformen dadurch ein, dass die Folie wenigstens teilweise zusammenfällt. Bei speziellen Ausführungsformen wird Qₙ teilweise durch eine Dehnung der Folie und teilweise durch Aufspannen der Folie durch ein Sprechluft-Volumenstrom von 5 l/min bei einem Umgebungsdruck von 1000 mbar erreicht.

Die Messung der Variabilität des Lumens eines von der Folie gebildeten Abschnitts der Sprechluft-Leitung unter verschiedenen Druckbedingungen kann in einer Materialprüfmaschine für Druckkraft erfolgen. Währenddessen kann der Druckabfall bei Durchströmung der Leitung messtechnisch ermittelt werden. Änderungen des Lumens machen sich dann deutlich über einen veränderten Druckabfall bemerkbar.

Bei bestimmten Ausführungsformen der vorliegenden Erfindung ist die Sprechluftzuführung zumindest abschnittsweise von einer Lage der Folie gebildet, die auf der Außenseite der Rohrwand des Kanülenrohrs befestigt ist. Bei einer alternativen Ausführungsform wird die Sprechluftzuführung zumindest abschnittsweise von einem aus der Folie bestehenden Schlauch gebildet, der auf der Außenseite der Rohrwand des Kanülenrohrs befestigt ist. Während bei der zuerst genannten Alternative das Lumen der Sprechluft-Leitung auf der einen Seite von der Lage der Folie und auf der anderen Seite von der Außenseite der Rohrwand des Kanülenrohrs definiert wird, wird bei der zuletzt genannten Alternative das Lumen der Sprechluftzuführung ausschließlich von dem aus der Folie bestehenden Schlauch definiert.

Bei bestimmten Ausführungsformen weisen die Folie oder der Schlauch eine vorgeformte Geometrie mit geringerem Querschnitt auf, wobei sich der vollständige Querschnitt erst dann ausbildet, wenn die Sprechluft das Lumen durchströmt. Ohne Sprechluftstrom nimmt die Folie oder der Schlauch wieder den Querschnitt der vorgeformten Geometrie ein. Die vorgeformte Geometrie kann beispielsweise eine oder mehrere in Längsrichtung verlaufende Einbuchtungen vorgeben, beispielweise durch unterschiedliche Wandstärken der Folie.

Die Folienlage bzw. der Folienschlauch werden bei bestimmten Ausführungsformen auf der Außenseite der Rohrwand des Kanülenrohrs durch Kleben oder Schweißen befestigt.

Die Aufbringung einer Lage der Folie auf die Außenseite der Rohrwand des Kanülenrohrs ist mit dem Vorteil verbunden, dass relativ wenig Folienmaterial verwendet werden muss. Zum anderen ergibt sich hierdurch eine typischerweise halbmondförmige Gestalt des Querschnitts des Lumens der Sprechluft-Leitung, wodurch ein Lumen erreicht werden kann, das in radialer Richtung relativ wenig aufträgt.

Die Anbringung der Sprechluft-Leitung in Form eines aus der Folie bestehenden Schlauches hat wiederum den Vorteil, dass ein solcher Schlauch an sich eine große Zuverlässigkeit im Hinblick auf die Dichtigkeit mit sich bringt, während im Falle der Anbringung einer Lage der Folie auf der Außenseite der Kanülenwand die Verbindung luftdicht erfolgen muss, um die erforderliche Dichtigkeit zu erreichen.

Bei handelsüblichen Kanälen für subglottische Absaugung wäre solch eine flexible Ausführung, wie sie hier beschrieben wird nicht zielführend, da ein weicher Absaugkanal beim Anlegen des für die Absaugung benötigten Vakuums kollabieren würde. Da eine effektive Absaugung im kollabierten Zustand nicht möglich wäre, müssen die Kanäle für die subglottische Absaugung zwangsläufig recht unflexible ausgeführt sein. Dies führt in vielen Fällen zu Druckstellen

Um sicherzustellen, dass die über die erfindungsgemäße Sprechluft-Leitung zugeführte Luft wie vorgesehen durch die natürlichen Atemwege entweicht, um auf diese Weise ACV zu ermöglichen, wird eine Ausführungsform der Erfindung vorgeschlagen, bei der die Sprechluft-Leitung im Stomaabschnitt zumindest abschnittsweise von einer aus der Folie bestehenden aufblasbaren Manschette gebildet wird, die sich ringförmig um das Kanülenrohr erstreckt und in Richtung Trachealabschnitt wenigstens einen Luftauslass aufweist. Diese aufblasbare Manschette kann dann, wenn diese von Sprechluft durchströmt wird, einen möglicherweise zwischen Tracheostomiekanüle und Stomagewebe bestehenden Spalt abdichten. Es hat sich gezeigt, dass insbesondere bei Patienten mit großem Stoma durch eine entsprechende Abdichtung die Qualität der Stimmerzeugung deutlich verbessert werden kann. Gerade Patienten, die dauerhaft eine Tracheostomiekanüle benötigen, haben häufig ein derart großes Stoma, dass der Spalt zwischen Stoma und Tracheostomiekanüle dazu führt, dass kein ausreichender Druck aufgebaut werden kann, um zu sprechen. Für diese Patienten ist eine ausreichende Abdichtung des Stomas Grundvoraussetzung, um überhaupt in irgendeiner Form phonieren zu können.

Bei einer bevorzugten Variante der eben beschriebenen Ausführungsform mit aufblasbarer Manschette weist die Manschette bei einem durchgeleiteten Luftstrom von 5 l/min im freien Zustand außerhalb eines Patienten einen Außendurchmesser auf, der um wenigstens 20 % größer ist als der Außendurchmesser des Kanülenrohrs auf Höhe der Manschette. Bei bestimmten Ausführungsformen der Erfindung weist die aufblasbare Manschette bei einem durchgeleiteten Luftstrom von 5 l/min einen Außendurchmesser auf, der um wenigstens 30 %, wenigstens 40 % oder gar wenigstens 50 % größer ist als der Außendurchmesser des Kanülenrohrs auf Höhe der Manschette.

Bei einer weiteren alternativen Ausführungsform der Erfindung wird die Sprechluft-Leitung sowohl im Stomaabschnitt als auch im Trachealabschnitt von einer Lage oder von einem Schlauch aus der Folie gebildet, wobei im Stomaabschnitt auf der Außenseite der Rohrwand des Kanülenrohrs zusätzlich eine Folie mit den erfindungsgemäßen Eigenschaften befestigt ist, wodurch sich eine aufblasbare Manschette ergibt, die sich ringförmig um das Kanülenrohr und über die Lage oder den Schlauch der Sprechluft-Leitung erstreckt, wobei die Lage oder der Schlauch in dem durch die aufblasbare Manschette verlaufenden Bereich, einen Luftauslass in das Lumen der Manschette aufweist. Strömt durch die Sprechluft-Leitung, die von der Lage oder dem Schlauch gebildet wird, Sprechluft, so bläst sich auch die darum liegende Manschette auf, da die Sprechluft über den in der Lage oder dem Schlauch im Bereich der Manschette vorgesehenen Luftauslass Sprechluft in das Lumen der Manschette strömt.

Bei einer Ausführungsform der Erfindung, bei der sich das Folienmaterial nicht wesentlich ausdehnt, sondern sich durch Faltenwurf an die Stomawand anschmiegt, sollte der Außendurchmesser der Manschette im voll aufgeblasenen freien Zustand wenigstens 20 %, vorzugsweise wenigstens 30 %, wenigstens 40 % oder wenigstens 50 % größer sein als der Außendurchmesser des Kanülenrohrs.

Bei bestimmten Ausführungsformen ist die Geometrie der Sprechluft-Leitung so gewählt, dass bei einem Sprechluftvolumenstrom von 5 l/min der hydraulische Durchmesser der Sprechluft-Leitung bei einem Umgebungsdruck von 1000 mbar mindestens 2,5 mm beträgt.

Der hydraulische Durchmesser des Luftaustritts sollte so dimensioniert sein, dass sich einerseits bei einem Luftstrom von 5 l/min durch diese Öffnung in der Manschette ein ausreichender Druck aufbaut, damit sich der Luftkanal derart an die Stomawand anschmiegt, dass eine ausreichende Abdichtung entsteht. Andererseits sollte der Druck nicht so groß sein, dass diese Abdichtung für den Patienten als schmerzhaft empfunden wird. Vorzugweise sollte der Anpressdruck auf das Gewebe nicht größer sein als 40 mbar. Da der maximale Druck lediglich für die kurze Zeit der Sprechluftzuführung stattfindet ist das Risiko von Druckstellen durch die Sprechluft-Leitung nahezu ausgeschlossen.

Je nach Ausführung weist der Querschnitt des Luftaustritts am distalen Ende der Sprechluft-Leitung einen vom Querschnitt der übrigen Sprechluft-Leitung abweichenden Querschnitt im Bereich von 1 bis 4,5 mm² auf.

Bei manchen Ausführungsformen weist die Folie der Sprechluft-Leitung eine besonders hohe Reißfestigkeit auf und ist wenigstens bis zu einem Sprechluftdruck von 200 mbar reißfest. Noch bevorzugter ist die Sprechluft-Leitung bis zu einem Sprechluftdruck von 500 mbar reißfest.

Die Reißfestigkeit und auch die Variabilität des Lumens der Sprechluft-Leitung werden zum Teil durch die Schichtdicke (Wandstärke) der Folie beeinflusst und teilweise durch das Material, aus dem die Folie besteht. Bei bestimmten Ausführungsformen der Erfindung beträgt die Wandstärke der Folie der Sprechluft-Leitung 5 bis 300 µm. Je dünner das Folienmaterial ist, desto anfälliger ist es typischerweise für Beschädigungen, wie z.B. Risse. Dickeres Folienmaterial trägt wiederum stärker auf und ist insofern von Nachteil. Es ist daher ein Abwägen erforderlich, welches Kriterium wie gewichtet werden soll.

Bei bestimmten Ausführungsformen beträgt die Wandstärke der Folie der Sprechluft-Leitung wenigstens 10 µm, wenigstens 20 µm oder wenigstens 30 µm. Vorzugsweise beträgt die Wandstärke bei bestimmten Ausführungsformen höchstens 250 µm oder höchstens 200 µm.

Bei bestimmten Ausführungsformen der Erfindung besteht die Folie der Sprechluft-Leitung aus einem Kunststoffpolymermaterial, das ausgewählt ist unter Polyurethan, Weich-PVC, PE, PP, EVA, PEBAX, Silikon.

Vorzugsweise hat die Folie der Sprechluft-Leitung bei bestimmten Ausführungsformen eine Bruchdehnung (nach DIN EN ISO 527) im Bereich von 150-600%.

Bei bestimmten Ausführungsformen besteht die Sprechluft-Leitung zumindest im Stomabereich aus einem Kunststoffpolymer mit einem Shore A im Bereich von 5-90.

Bei bestimmten Ausführungsformen weist die erfindungsgemäße Beatmungsvorrichtung zusätzlich eine separate Absaugleitung zum Absaugen von subglottischem Sekret auf.

Bei den Ausführungsformen, bei denen eine separate Absaugleitung zum Absaugen von subglottischem Sekret vorgesehen ist, kann diese entweder in der Wand der Tracheostomiekanüle eingebracht sein oder auf der Außenseite der Rohrwand befestigt sein. Im letzteren Fall verläuft die Folie der Sprechluft-Leitung auf der Oberseite der Absaugleitung seitlich neben der Absaugleitung. Bei einer alternativen Ausführungsform überspannt die Folie der Sprechluft-Leitung die Absaugleitung in Längsrichtung wenigstens abschnittsweise.

Vorzugsweise ist der Luftauslass der Sprechluft-Leitung in proximaler Richtung in einer Entfernung von wenigstens 2 mm vom Sekreteinlass der Absaugleitung angeordnet. Dieser Mindestabstand gewährleistet, dass die Luft zum Sprechen nicht durch eine Restmenge an verbliebenem Sekret durchströmen muss. Dies würde die Sprachqualität verschlechtern und hätte zudem auch hygienische Nachteile, da sich Aerosole bilden könnten.

Bei bestimmten Ausführungsformen der Erfindung ist an dem proximalen Ende der Sprechluft-Leitung ein Verbindungsstück für den Anschluss an eine Druckluftquelle vorgesehen, wie z.B. ein Y-Stück oder ein Konnektor. Bei bestimmten Ausführungsformen weist das Verbindungsstück ein Ventil, einen Regler und/oder eine mit einem Finger verschließbare Öffnung auf (=Fingertip-Konnektor). Hierüber kann gesteuert werden, wann und ggf. auch wieviel Druckluft über die Sprechluft-Leitung in den subglottischen Bereich einströmt.

Bestimmte Ausführungsformen der Erfindung sind dadurch gekennzeichnet, dass die Sprechluft-Leitung im Bereich des Extrakorporalabschnitts aus einem flexiblen Kunststoffschlauch besteht, der eine größere Härte und/oder Wandstärke aufweist, als die Folie, aus der die Sprechluftzuführung zumindest abschnittsweise im Stomaabschnitt und/oder Trachealabschnitt besteht.

Bei bestimmten Ausführungsformen der Erfindung weist die Sprechluft-Leitung am Luftauslass an deren distalem Ende zusätzlich noch ein Ventil auf. Erfindungsgemäß sollte dieses Ventil so eingerichtet sein, dass es den Luftaustritt in den subglottischen Bereich erlaubt, während es den Lufteintritt aus dem subglottischen Bereich in die Sprechluft-Leitung verhindert. Die Ventilfunktion ermöglicht es, Unterdruck in der Sprechluft-Leitung zu erzeugen, um erforderlichenfalls den Querschnitt des Lumens der Sprechluft-Leitung durch Anlegen von Unterdruck gezielt zu reduzieren.

Bei bestimmten Ausführungsformen wird die Ventilfunktion durch ein Klappenventil bereitgestellt. Bei alternativen Ausführungsform wird die Ventilfunktion dadurch bereitgestellt, dass der letzte Abschnitt des Luftaustritts im Vergleich zu den übrigen Abschnitten der Sprechluft-Leitung deutlich dünnwandiger ausgestaltet ist, sodass sich dieser Abschnitt bei Anlegen eines Unterdruckes verschließt. Vorzugsweise beträgt die Länge des besonders dünnwandig ausgestalteten Abschnitts des Luftaustritts 0,3-1 cm oder mehr.

Für Zwecke der ursprünglichen Offenbarung wird darauf hingewiesen, dass sämtliche Merkmale, wie sie sich aus der vorliegenden Beschreibung, den anhängenden Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

In den anhängenden Figuren und der dazugehörigen Figurenbeschreibung sind einzelne konkrete Ausführungsformen der Erfindung schematisch dargestellt. Diese konkreten Ausführungsformen sind lediglich ein Beispiel für mögliche Merkmalskombinationen. Die Erfindung ist jedoch keinesfalls auf diese konkreten Ausführungsformen beschränkt, sondern umfasst alle vom Schutzumfang der Patentansprüche abgedeckten Ausführungsformen, auch wenn sie nicht explizit dargestellt oder beschrieben sind.

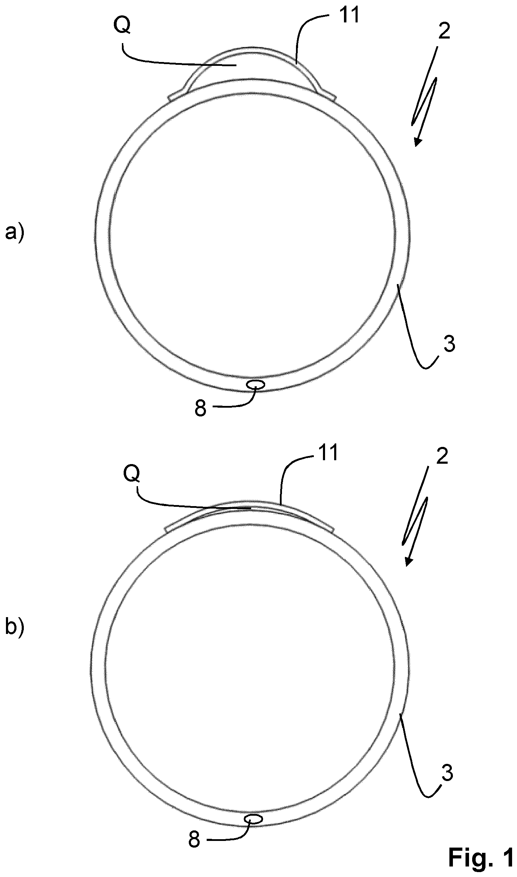

- Figur 1:: Die Abbildungen a) und b) von Figur 1 stellen einen schematischen Querschnitt durch das Kanülenrohr im Trachealabschnitt einer Ausführungsform einer erfindungsgemäßen Beatmungsvorrichtung dar, bei der die Sprechluft-Leitung in Form einer auf der Außenseite des Kanülenrohres angeordneten Folienlage realisiert ist, wobei in der Abbildung a) der Zustand bei einem Sprechluft-Volumenstrom von 5 l/min bei normalem Umgebungsdruck dargestellt ist.

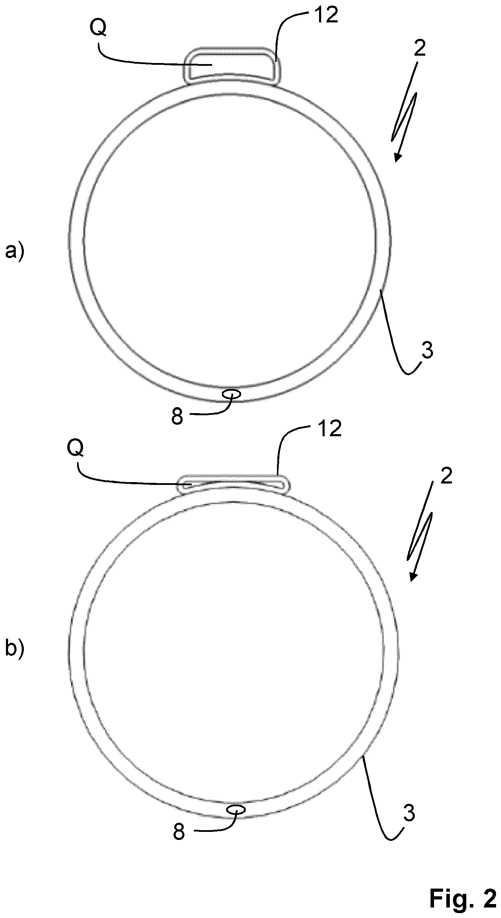

- Figur 2:: Die Abbildungen a) und b) von Figur 2 stellen einen schematischen Querschnitt durch das Kanülenrohr im Trachealabschnitt einer Ausführungsform einer erfindungsgemäßen Beatmungsvorrichtung dar, bei der die Sprechluft-Leitung in Form eines auf der Außenseite des Kanülenrohres angeordneten Folienschlauchs realisiert ist, wobei in der Abbildung a) der Zustand bei einem Sprechluft-Volumenstrom von 5 l/min bei normalem Umgebungsdruck dargestellt ist.

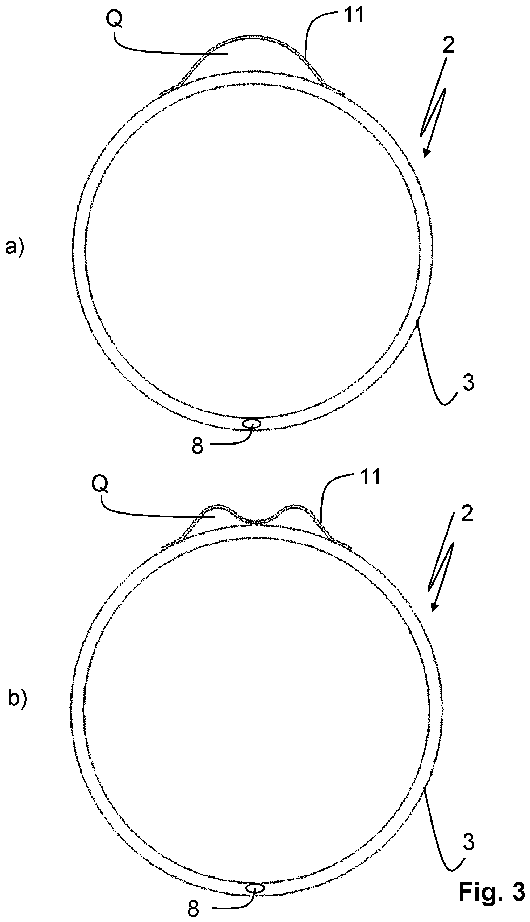

- Figur 3:: Die Abbildungen a) und b) von Figur 3 stellen einen schematischen Querschnitt durch das Kanülenrohr im Trachealabschnitt einer Ausführungsform einer erfindungsgemäßen Beatmungsvorrichtung dar, bei der die Sprechluft-Leitung in Form einer auf der Außenseite des Kanülenrohres angeordneten vorgespannten Folienlage realisiert ist, wobei in der Abbildung a) der Zustand bei einem Sprechluft-Volumenstrom von 5 l/min bei normalem Umgebungsdruck dargestellt ist.

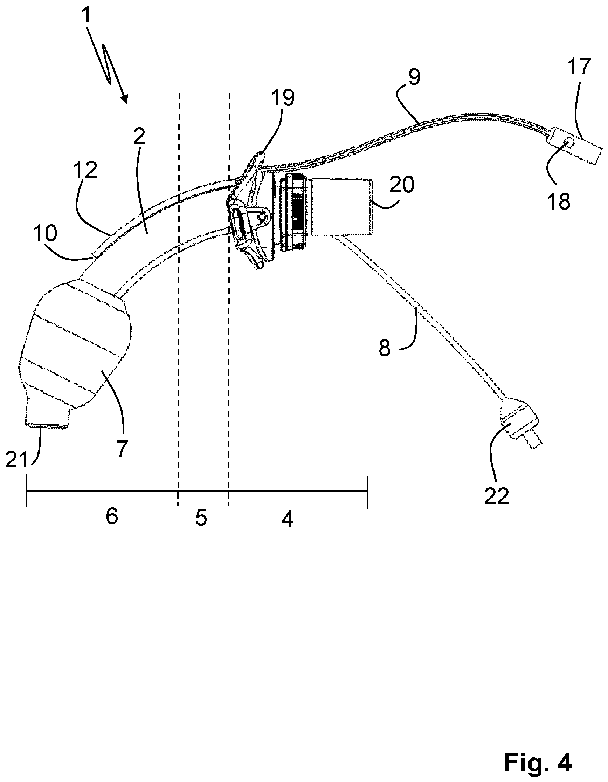

- Figur 4:: Figur 4 zeigt eine Draufsicht von der Seite auf eine Ausführungsform einer erfindungsgemäßen Beatmungsvorrichtung, bei der die Sprechluft-Leitung im Stomaabschnitt und im Trachealabschnitt in Form eines Folienschlauches vorgesehen ist.

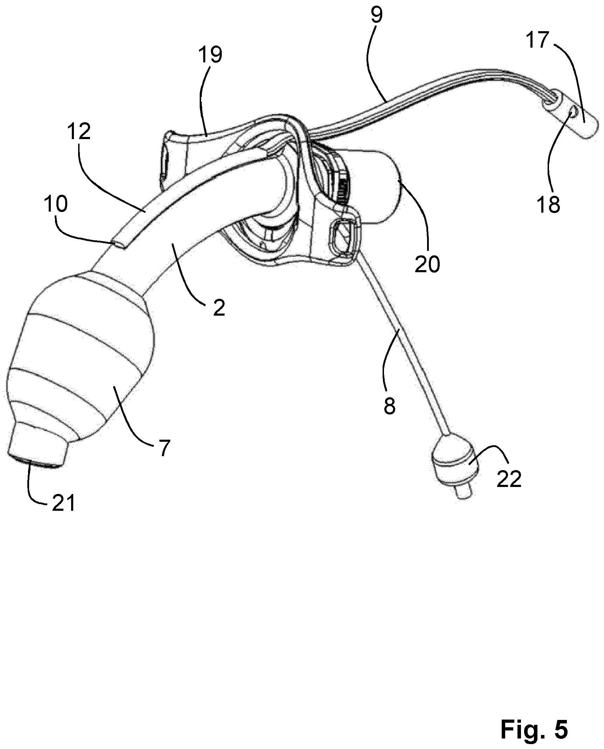

- Figur 5:: Figur 5 zeigt eine perspektivische Ansicht auf eine Ausführungsform einer erfindungsgemäßen Beatmungsvorrichtung, bei der die Sprechluft-Leitung im Stomaabschnitt und im Trachealabschnitt in Form eines Folienschlauches vorgesehen ist.

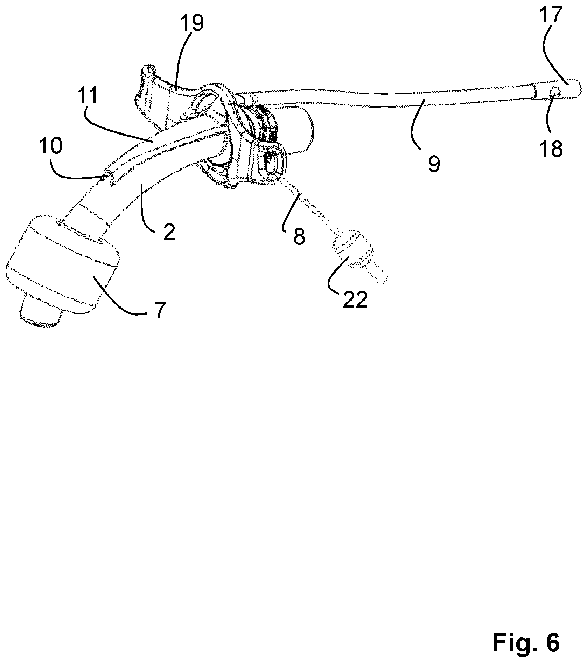

- Figur 6:: Figur 6 zeigt eine perspektivische Ansicht auf eine Ausführungsform einer erfindungsgemäßen Beatmungsvorrichtung, bei der die Sprechluft-Leitung im Stomaabschnitt und im Trachealabschnitt in Form einer Folienlage vorgesehen ist.

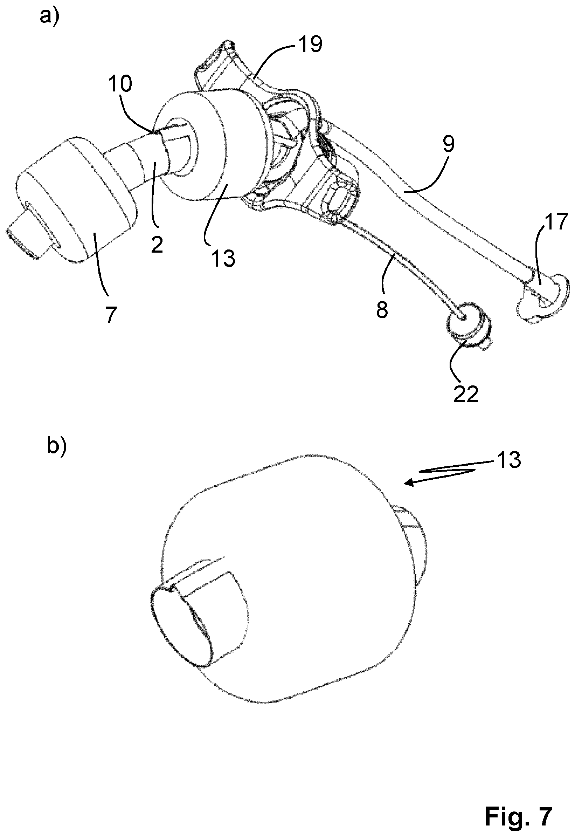

- Figur 7:: Abbildung a) von Figur 7 zeigt eine perspektivische Ansicht auf eine Ausführungsform einer erfindungsgemäßen Beatmungsvorrichtung, bei der die Sprechluft-Leitung im Stomaabschnitt und im Trachealabschnitt in Form einer Folienmanschette vorgesehen ist. Abbildung b) von Figur 7 zeigt die separate Folienmanschette, bevor sie auf das Kanülenrohr gefügt wurde.

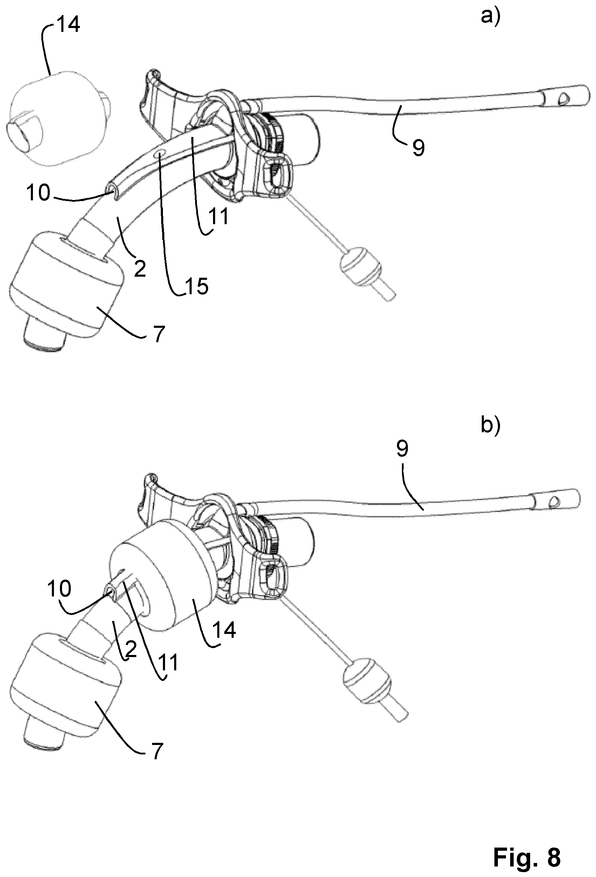

- Figur 8:: Figur 8 zeigt eine perspektivische Ansicht auf eine Ausführungsform einer erfindungsgemäßen Beatmungsvorrichtung, bei der die Sprechluft-Leitung im Stomaabschnitt und im Trachealabschnitt aus Kombination einer Folienlage und einer Folienmanschette vorgesehen ist, wobei Abbildung a) den Zustand ohne montierte Folienmanschette darstellt und Abbildung b) den Zustand mit montierter Folienmanschette darstellt.

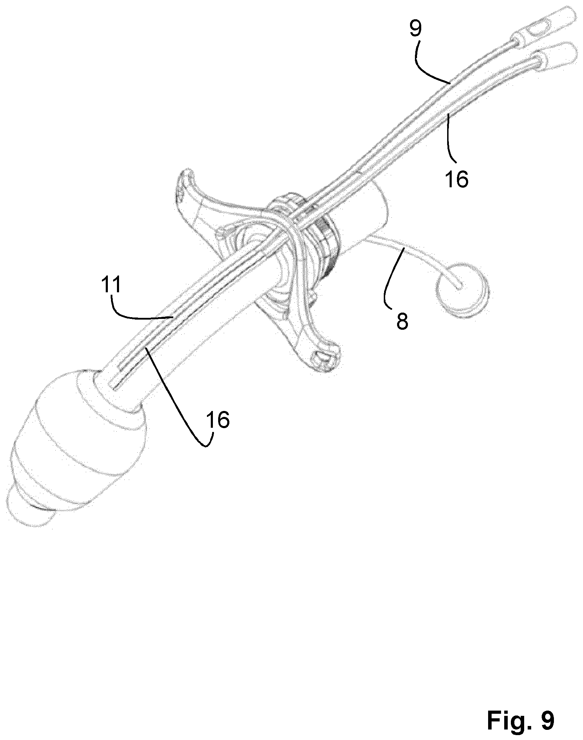

- Figur 9:: In Figur 9 ist eine Ausführungsform einer erfindungsgemäßen Beatmungsvorrichtung dargestellt, die neben der Sprechluft-Leitung auch eine subglottische Absaugleitung aufweist.

Figur 1 zeigt einen schematischen Querschnitt durch das Kanülenrohr 2 einer Ausführungsform einer erfindungsgemäßen Beatmungsvorrichtung im Trachealabschnitt. Die Rohrwand 3 des Kanülenrohres definiert in diesem Bereich einen kreisrunden Kanülenrohrinnenraum durch den die Atemluft strömen kann. Auf der Außenseite des Kanülenrohres 2 ist auf der Rohrwand 3 eine flexible Kunststofffolienlage 11 angeordnet, die bei einem Sprechluftvolumen von 5 l/min in dem hier dargestellten Abschnitt ein Lumen mit einem Querschnitt Q definiert (Abbildung a)). Die Cuffluft-Leitung 8 verläuft bei der hier dargestellten Ausführungsform in der Rohrwand 3. In der Abbildung b) ist der Zustand dargestellt, in dem keine Sprechluft mehr durch die Sprechluft-Leitung geführt wird, wodurch sich der Querschnitt des Lumens das zwischen der Folienlage 11 und der Außenseite der Rohrwand 3 um über 50% reduziert.

Figur 2 zeigt einen schematischen Querschnitt durch das Kanülenrohr 2 einer Ausführungsform einer erfindungsgemäßen Beatmungsvorrichtung im Trachealabschnitt. Die Rohrwand 3 des Kanülenrohres definiert in diesem Bereich einen kreisrunden Kanülenrohrinnenraum durch den die Atemluft strömen kann. Auf der Außenseite des Kanülenrohres 2 ist auf der Rohrwand 3 eine flexibler Folienschlauch 12 angeordnet, der bei einem Sprechluftvolumen von 5 l/min in dem hier dargestellten Abschnitt ein Lumen mit einem Querschnitt Q definiert (Abbildung a)). Die Cuffluft-Leitung 8 verläuft bei der hier dargestellten Ausführungsform in der Rohrwand 3. In der Abbildung b) ist der Zustand dargestellt, in dem keine Sprechluft mehr durch die Sprechluft-Leitung geführt wird, wodurch sich der Querschnitt des Lumens das zwischen dem Folienschlauch 12 und der Außenseite der Rohrwand 3 um über 50% reduziert.

Figur 3 zeigt einen schematischen Querschnitt durch das Kanülenrohr 2 einer Ausführungsform einer erfindungsgemäßen Beatmungsvorrichtung im Trachealabschnitt. Die Rohrwand 3 des Kanülenrohres definiert in diesem Bereich einen kreisrunden Kanülenrohrinnenraum durch den die Atemluft strömen kann. Auf der Außenseite des Kanülenrohres 2 ist auf der Rohrwand 3 eine flexibler Folienschlauch 12 angeordnet, der bei einem Sprechluftvolumen von 5 l/min in dem hier dargestellten Abschnitt ein Lumen mit einem Querschnitt Q definiert (Abbildung a)). Die Cuffluft-Leitung 8 verläuft bei der hier dargestellten Ausführungsform in der Rohrwand 3. In der Abbildung b) ist der Zustand dargestellt, in dem keine Sprechluft mehr durch die Sprechluft-Leitung geführt wird, wodurch sich der Querschnitt des Lumens das zwischen dem Folienschlauch 12 und der Außenseite der Rohrwand 3 um über 30% reduziert.

In der Draufsicht von der Seite auf eine Ausführungsform der erfindungsgemäßen Beatmungsvorrichtung, die in Figur 4 dargestellt ist, kann man erkennen, dass der Extrakorporalabschnitt 4 der Bereich ist, der sich ab dem Kanülenschild 19 in proximaler Richtung bis zum proximalen Ende der Kanüle erstreckt. In distaler Richtung schließt sich an den Kanülenschild 19 zunächst der Stomaabschnitt 5 an, direkt gefolgt von dem Trachealabschnitt 6. Der Stomaabschnitt 5 ist ein standardisierter Bereich, der je nach Größe und Halsdurchmesser des Patienten etwas länger (Erwachsene) oder etwas kürzer (Kinder) ausgestaltet ist. Dementsprechend variiert auch die Länge des Trachealabschnitts 6, der sich in distaler Richtung unmittelbar an den Stomaabschnitt 5 anschließt.

Die in Figur 4 dargestellte Beatmungsvorrichtung 1 weist neben der Sprechluft-Leitung 9 auch eine Cuffluft-Leitung 8 auf. Die Cuffluft-Leitung 8 weist an ihrem proximalen Ende einen Kontrollballon 22 auf und im Stoma- und Trachealabschnitt verläuft die Cuffluft-Leitung 8 auf der Unterseite des Kanülenrohres 2 bis zum Cuff 7, in welchen die Cuffluft-Leitung 8 mit einem Luftauslass (nicht dargestellt) mündet. Das Kanülenrohr 2 weist eine proximale Atemluftöffnung 20 und eine distale Atemluftöffnung 21 auf, über die die Atemluft in das Lumen des Kanülenrohres 2 ein- und ausströmen kann. Die Sprechluft-Leitung 9 weist an deren proximalem Ende einen Konnektor 17 auf, über den die Verbindung mit einer Druckluftquelle hergestellt werden kann. Durch Abdecken bzw. Freigeben der Ventilöffnung 18 kann der Patient steuern ob ein kontinuierlich bereitgestellter Druckluftstrom über den extrakorporalen Abschnitt der Sprechluft-Leitung 9 strömt oder den Weg des geringsten Widerstandes durch die Ventilöffnung 18 nimmt. Die über den extrakorporalen Abschnitt der Sprechluft-Leitung 9 geführte Sprechluft tritt in distaler Richtung hinter dem Kanülenschild 19 in den Stomaabschnitt 5 der Sprechluft-Leitung 9 und anschließend in den Trachealabschnitt 6 der Sprechluft-Leitung 9 ein. Im Trachealabschnitt 6 mündet die Sprechluft-Leitung 9 schließlich in einem Luftauslass 10 in einem Bereich zwischen dem Stomaabschnitt und dem Cuff. Im Stomaabschnitt 5 und im Trachealabschnitt 10 ist die Sprechluft-Leitung 9 in Form eines auf der Oberseite des Kanülenrohres 2 befestigten Folienschlauches 12 vorgesehen.

In Figur 5 ist die Ausführungsform von Figur 4 noch einmal in einer perspektivischen Ansicht dargestellt. Hier ist insbesondere der Übergang des extrakorporalen Bereichs der Sprechluft-Leitung 9 in den Folienschlauch 12 gut zu erkennen. Dieser Übergang findet hinter der Ringhalterung 19 an der Stelle statt, an welcher sich der Stomaabschnitt des Kanülenrohres an den Extrakorporalabschnitt des Kanülenrohres anschließt (vgl. Figur 4). Der Extrakorporalabschnitt der Sprechluft-Leitung 9 besteht aus einem stabileren Schlauchmaterial im Vergleich zu dem Folienschlauch 12, der im intrakorporalen Abschnitt vorgesehen ist. Dies ist insbesondere dem Umstand geschuldet, dass außerhalb des Körpers größere mechanische Belastungen auf die Sprechluft-Leitung 9 einwirken können, denen es hier somit Stand zu halten gilt.

In Figur 6 ist eine Ausführungsform der erfindungsgemäßen Beatmungsvorrichtung dargestellt, bei der die Sprechluft-Leitung im Stomaabschnitt und im Extrakorporalabschnitt in Form einer Folienlage 11 vorgesehen ist, die im Trachealabschnitt einen Luftauslass 10 aufweist. Die Folienlage ist so auf dem Kanülenrohr 2 aufgebracht, dass sich im hier dargestellten Betrieb mit einem Sprechluft-Volumenstrom von 5 l/min ein tunnelförmiges Lumen ausbildet.

In Figur 7 ist eine spezielle Ausführungsform einer erfindungsgemäßen Beatmungsvorrichtung dargestellt, bei welcher die Sprechluft-Leitung 9 im Stomaabschnitt in Form einer Folienmanschette 13 vorgesehen ist. Die Folienmanschette 13 weist in distaler Richtung einen Luftauslass 10 auf, der bei der hier dargestellten Ausführungsform noch ein Stück weit bis in den Trachealabschnitt hinein ragt. In der Abbildung a) ist der montierte Zustand dargestellt, in dem die Manschette 13 ringförmig um das Kanülenrohr 2 angeordnet ist. In der Abbildung b) ist die ringförmige Manschette 13 mit dem daran angeordneten Luftauslass separat, d.h. im nicht montierten Zustand dargestellt.

In Figur 8 ist eine weitere besondere Ausführungsform der erfindungsgemäßen Beatmungsvorrichtung dargestellt, bei der die Sprechluft-Leitung in Form einer auf der Außenseite des Kanülenrohres aufgebrachten Folienlage 11 vorgesehen ist. Diese Folienlage 11 erstreckt sich bis zum Luftauslass 10 in den Trachealabschnitt. Zusätzlich zum Luftauslass 10 im Trachealabschnitt weist die Folienlage 11 im Stomaabschnitt einen weiteren Luftauslass 15 auf, der in das Lumen einer ringförmige Manschetten 14 mündet, die um das Kanülenrohr 2 angeordnet ist uns gleichzeitig auch die Folienlage 11 der Sprechluft-Leitung im Stomaabschnitt umfasst. Strömt Sprechluft durch die Sprechluft-Leitung 9 und gelangt im Stomaabschnitt in das Lumen das sich zwischen der Folienlage 11 und der Außenseite der Rohrwand des Kanülenrohres 2 aufspannt, kann ein Teil der Sprechluft über den zusätzlichen Luftauslass 15 im Stomaabschnitt des Folienschlauches 11 in das Lumen der zusätzlichen Manschette 14 gelangen, wodurch die Manschette 14 aufgeblasen wird. Auf diese Weise kann über den Sprechluftvolumenstrom temporär eine Abdichtung des Stomas während des Sprechvorgangs erreicht werden.

In der Abbildung b) von Figur 8 ist der Zustand dargestellt, in dem die zusätzliche Manschette 14 ringförmig um das Kanülenrohr 2 und die Folienlage 11 der Sprechluft-Leitung 9 montiert ist. In Abbildung a) ist die zusätzliche Manschette 14 dargestellt, damit der zusätzliche Luftauslass 15 im Folienschlauch 11 zu erkennen ist.

In Figur 9 ist eine Ausführungsform einer erfindungsgemäßen Beatmungsvorrichtung dargestellt, bei der zusätzlich zu der Cuffluft-Leitung 8 und der Sprechluft-Leitung 9 noch eine Absaugleitung 16 für subglottisches Sekret vorgesehen ist. Die Absaugleitung 16 läuft ab dem Stomaabschnitt in distaler Richtung im Wesentlichen parallel und neben der Folienlage 11 der Sprechluft-Leitung 9 bis in den Trachealabschnitt. Im Trachealabschnitt reicht die Absaugleitung 16 jedoch etwas weiter als die Folienlage 11 der Sprechluft-Leitung 9. Der Sekreteintritt in die Absaugleitung 16 sollte möglichst nah am Cuff angebracht sein, damit möglichst viel Sekret abgesaugt wird. Für den Fall, dass dennoch etwas Sekret oberhalb des Cuffs vorhanden ist, sollte die Luftaustritt der Sprechluft-Leitung 11 etwas weiter proximal versetzt sein. Auf diese Weise soll sichergestellt werden, dass die Luft zum Sprechen nicht durch einen eventuell nach der Absaugung noch vorhandenem Sekretrückstand durchgeleitet wird. Die würde die Sprachqualität verschlechtern und hätte zudem auch hygienische Nachteile.. Da beim Absaugen erheblicher Unterdruck angelegt werden muss, besteht die Absaugleitung 16 aus einem wesentlich stabileren Schlauchmaterial als der Folienschlauch 11 der Sprechluft-Leitung 9.

### Bezugszeichen

- 1: Beatmungsvorrichtung
- 2: Kanülenrohr
- 3: Rohrwand
- 4: Extrakorporalabschnitt
- 5: Stomaabschnitt
- 6: Trachealabschnitt
- 7: Cuff
- 8: Cuffluft-Leitung
- 9: Sprechluft-Leitung
- 10: Luftauslass
- 11: Lage
- 12: Schlauch
- 13, 14: Manschette
- 15: Luftauslass
- 16: Absaugleitung
- 17: Konnektor
- 18: Ventil
- 19: Kanülenschild
- 20: proximale Atemluftöffnung
- 21: distale Atemluftöffnung
- 22: Kontrollballon

## Patentansprüche

1. Beatmungsvorrichtung (1), insbesondere Tracheostomiekanüle oder Endotrachealtubus, mit
▪ einem Kanülenrohr (2), dessen Rohrwand (3) einen Extrakorporalabschnitt (4), einen Stomaabschnitt (5) und einen Trachealabschnitt (6) aufweist,
▪ einem aufblasbaren Cuff (7), der sich im Trachealabschnitt (6) an der Außenseite der Rohrwand (3) ringförmig um das Kanülenrohr (2) erstreckt,
▪ einer Cuffluft-Leitung (8), die einen Luftauslass im Cuff aufweist,
▪ einer Sprechluft-Leitung (9), die einen Luftauslass (10) in dem Bereich zwischen Stomaabschnitt (5) und Cuff (7) aufweist,
**dadurch gekennzeichnet, dass** die Sprechluft-Leitung (9) im Bereich des Stomaabschnitts (5) und/oder des Trachealabschnitts (6) zumindest abschnittsweise von einer auf der Außenseite des Kanülenrohres (2) angeordneten Folie (11, 12, 13, 14) gebildet wird, die ein variables Lumen für die Durchführung von Sprechluft ausbildet, wobei
- das variable Lumen eines von der Folie (11, 12, 13, 14) gebildeten Abschnitts n der Sprechluft-Leitung (9) bei einem Sprechluft-Volumenstrom von 5 Litern pro Minute und einem Umgebungsdruck von 1000 mbar einen Querschnitt Qₙ aufweist,
- der Querschnitt des Lumens des Abschnitts n bei einem auf die Außenseite der Folie wirkenden Überdruck von bis zu 15 mbar um wenigstens 30% im Vergleich zum Querschnitt Qₙ reduziert wird, wenn keine Sprechluft durch die Sprechluft-Leitung strömt.

2. Vorrichtung nach Anspruch 1, **dadurch gekennzeichnet, dass** die Sprechluft-Leitung zumindest abschnittsweise von einer Lage (11) der Folie gebildet wird, die auf der Außenseite der Rohrwand des Kanülenrohres angeordnet ist.

3. Vorrichtung nach Anspruch 1, **dadurch gekennzeichnet, dass** die Sprechluft-Leitung zumindest abschnittsweise von einem aus der Folie bestehenden Schlauch (12) gebildet wird, der auf der Außenseite der Rohrwand des Kanülenrohres angeordnet ist.

4. Vorrichtung nach Anspruch 1, **dadurch gekennzeichnet, dass** die Sprechluft-Leitung im Stomaabschnitt zumindest abschnittsweise von einer aus der Folie bestehenden aufblasbaren Manschette (13) gebildet wird, die sich ringförmig um das Kanülenrohr erstreckt und in Richtung Trachealabschnitt wenigstens einen Luftauslass (10) aufweist.

5. Vorrichtung nach Anspruch 4, **dadurch gekennzeichnet, dass** die aufblasbare Manschette bei einem durchgeleiteten Luftstrom von 5 Litern pro Minute einen Außendurchmesser aufweist, der um wenigstens 20% größer ist als der Außendurchmesser des Kanülenrohres auf Höhe der Manschette.

6. Vorrichtung nach einem der Ansprüche 1 bis 3, **dadurch gekennzeichnet, dass** die Sprechluft-Leitung sowohl im Stomaabschnitt als auch abschnittsweise im Trachealabschnitt von einer Lage oder von einem Schlauch aus der Folie gebildet wird, wobei im Stomaabschnitt auf der Außenseite der Rohrwand des Kanülenrohres eine zusätzliche aufblasbare Manschette (14) aus der Folie vorgesehen ist, die sich ringförmig um das Kanülenrohr und über die Lage oder den Schlauch der Sprechluft-Leitung erstreckt, wobei die Lage oder der Schlauch in dem durch die aufblasbare Manschette verlaufenden Bereich, einen zusätzlichen Luftauslass (15) in das Lumen der Manschette aufweist.

7. Vorrichtung nach einem der Ansprüche 1 bis 6, **dadurch gekennzeichnet, dass** bei einem Sprechluft-Volumenstrom von 5 Litern pro Minute der hydraulische Durchmesser der Sprechluft-Leitung bei einem Umgebungsdruck von 1000 mbar mindestens 2,5 mm beträgt.

8. Vorrichtung nach einem der Ansprüche 1 bis 7, **dadurch gekennzeichnet, dass** die Folie der Sprechluft-Leitung wenigstens bis zu einem Sprechluft-Druck von 200 mbar reißfest ist.

9. Vorrichtung nach einem der Ansprüche 1 bis 8, **dadurch gekennzeichnet, dass** die Folie der Sprechluft-Leitung eine Wandstärke im Bereich von 5 bis 300 µm aufweist.

10. Vorrichtung nach einem der Ansprüche 1 bis 9, **dadurch gekennzeichnet, dass** die Folie der Sprechluft-Leitung zumindest im Stomabereich aus einem Kunststoffpolymer mit einem Shore A im Bereich von 5 bis 90 besteht.

11. Vorrichtung nach einem der Ansprüche 1 bis 10, **dadurch gekennzeichnet, dass** diese zusätzlich eine separate Absaugleitung (16) zum Absaugen von subglottischem Sekret aufweist.

12. Vorrichtung nach Anspruch 11, **dadurch gekennzeichnet, dass** die Absaugleitung zum Absaugen von subglottischem Sekret an der Außenseite der Rohrwand des Kanülenrohres befestigt ist, wobei die Folie der Sprechluft-Leitung auf der Oberseite der Absaugleitung oder seitlich neben der Absaugleitung verläuft oder die Absaugleitung in Längsrichtung wenigstens abschnittsweise überspannt.

13. Vorrichtung nach einem der Ansprüche 1 bis 12, **dadurch gekennzeichnet, dass** die Sprechluft-Leitung an deren proximalem Ende einen Konnektor (17) für Druckluft, vorzugsweise einen Fingertip-Konnektor, aufweist.

14. Vorrichtung nach einem der Ansprüche 1 bis 13, **dadurch gekennzeichnet, dass** die Sprechluft-Leitung im Bereich des Extrakorporalabschnitts aus einem flexiblen Kunststoffschlauch besteht, der eine größere Härte und/oder Wandstärke aufweist, als die Folie, aus der die Sprechluft-Leitung zumindest abschnittsweise im Stomaabschnitt und/oder Trachealabschnitt besteht.

15. Vorrichtung nach einem der Ansprüche 1 bis 14, **dadurch gekennzeichnet, dass** die Sprechluft-Leitung am Luftauslass an deren distalem Ende ein Ventil (18) aufweist.

16. Vorrichtung nach einem der Ansprüche 1 bis 15, **dadurch gekennzeichnet, dass** sich der Querschnitt des Lumens des Abschnitts n bei einem auf die Außenseite der Folie wirkenden Überdruck von bis zu 15 mbar um höchstens 10% im Vergleich zum Querschnitt Qₙ reduziert, wenn durch die Sprechluft-Leitung Sprechluft mit einem Sprechluft-Volumenstrom von 5 Litern pro Minute strömt.

## Claims

1. Ventilation device (1), in particular a tracheostomy cannula or an endotracheal tube, comprising
• a cannula tube (2), of which a tube wall (3) has an extracorporeal portion (4), a stoma portion (5) and a tracheal portion (6),
• an inflatable cuff (7), which extends in the tracheal portion (6) annularly about the cannula tube (2), on the outer side of the tube wall (3),
• a cuff air line (8), which has an air outlet in the cuff,
• a voice air line (9), which has an air outlet (10) in the region between stoma portion (5) and cuff (7),
**characterized in that** the voice air line (9), in the region of the stoma portion (5) and/or of the tracheal portion (6), is formed at least in part by a film (11, 12, 13, 14) arranged on the outer side of the cannula tube (2), which film defines a variable lumen for the passage of voice air, wherein
- the variable lumen of a portion n of the voice air line (9) formed by the film (11, 12, 13, 14) has, at a voice air volumetric flow of 5 litres per minute and at an ambient pressure of 1000 mbar, a cross section Qₙ,
- with an overpressure of up to 15 mbar acting on the outer side of the film, the cross section of the lumen of the portion n is reduced by at least 30%, compared to the cross section Qₙ, when no voice air flows through the voice air line.

2. Device according to Claim 1, **characterized in that** the voice air line is formed at least in part by a layer (11) of the film which is arranged on the outer side of the tube wall of the cannula tube.

3. Device according to Claim 1, **characterized in that** the voice air line is formed at least in part by a hose (12) consisting of the film, which hose is arranged on the outer side of the tube wall of the cannula tube.

4. Device according to Claim 1, **characterized in that** the voice air line in the stoma portion is formed at least in part by an inflatable sleeve (13) consisting of the film, which sleeve extends annularly around the cannula tube and has at least one air outlet (10) in the direction of the tracheal portion.

5. Device according to Claim 4, **characterized in that**, with an air flow of 5 litres per minute passing through, the inflatable sleeve has an external diameter that is at least 20% greater than the external diameter of the cannula tube at the level of the sleeve.

6. Device according to one of Claims 1 to 3, **characterized in that** the voice air line is formed, both in the stoma portion and in part in the tracheal portion, by a layer or by a hose made of the film, wherein an additional inflatable sleeve (14) made of the film is provided in the stoma portion, on the outer side of the tube wall of the cannula tube, which additional inflatable sleeve extends annularly around the cannula tube and over the layer or the hose of the voice air line, wherein the layer or the hose in the region running through the inflatable sleeve has an additional air outlet (15) into the lumen of the sleeve.

7. Device according to one of Claims 1 to 6, **characterized in that**, with a voice air volumetric flow of 5 litres per minute, the hydraulic diameter of the voice air line is at least 2.5 mm at an ambient pressure of 1000 mbar.

8. Device according to one of Claims 1 to 7, **characterized in that** the film of the voice air line is resistant to tearing, at least up to a voice air pressure of 200 mbar.

9. Device according to one of Claims 1 to 8, **characterized in that** the film of the voice air line has a wall thickness in the range from 5 to 300 µm.

10. Device according to one of Claims 1 to 9, **characterized in that** the film of the voice air line consists, at least in the stoma region, of a plastic polymer with a Shore A in the range from 5 to 90.

11. Device according to one of Claims 1 to 10, **characterized in that** it additionally has a separate suction line (16) for aspiration of subglottic secretions.

12. Device according to Claim 11, **characterized in that** the suction line for the aspiration of subglottic secretions is fastened to the outer side of the tube wall of the cannula tube, wherein the film of the voice air line runs on the top of the suction line or laterally alongside the suction line or at least in part spans the suction line in the longitudinal direction.

13. Device according to one of Claims 1 to 12, **characterized in that** the voice air line has, at its proximal end, a connector (17) for compressed air, preferably a fingertip connector.

14. Device according to one of Claims 1 to 13, **characterized in that** the voice air line, in the region of the extracorporeal portion, consists of a flexible plastic hose which has a greater hardness and/or wall thickness than the film from which the voice air line is made, at least in part, in the stoma portion and/or tracheal portion.

15. Device according to one of Claims 1 to 14, **characterized in that** the voice air line has a valve (18) at the air outlet at its distal end.

16. Device according to one of Claims 1 to 15, **characterized in that**, with an overpressure of up to 15 mbar acting on the outer side of the film, the cross section of the lumen of the portion n is reduced by at most 10%, compared to the cross section Qₙ, when voice air flows through the voice air line at a voice air volumetric flow of 5 litres per minute.

## Revendications

1. Dispositif de ventilation (1), notamment tube de trachéotomie ou tube endotrachéal, comprenant
▪ un tube de canule (2) dont la paroi de tube (3) comporte une portion extracorporelle (4), une portion de stomie (5) et une portion trachéale (6),
▪ un brassard gonflable (7) qui s'étend annulairement autour du tube de canule (2) dans la portion trachéale (6) sur le côté extérieur de la paroi de tube (3),
▪ un conduit d'air de brassard (8) qui comporte une sortie d'air dans le brassard,
▪ un conduite d'air vocal (9) qui comporte une sortie d'air (10) dans la zone située entre la portion de stomie (5) et le brassard (7),
**caractérisé en ce que** le conduit d'air vocal (9) dans la zone de la portion de stomie (5) et/ou de la portion trachéale (6) est formé au moins par portions par un film (11, 12, 13, 14) disposé sur le côté extérieur du tube de canule (2) et formant une lumière variable destinée à conduire l'air vocal,
- la lumière variable d'une portion n du conduit d'air vocal (9) formée par le film (11, 12, 13, 14) ayant une section transversale Qn pour un débit volumique d'air vocal de 5 litres par minute et une pression ambiante de 1000 mbar,
- la section transversale de la lumière de la portion n étant réduite d'au moins 30 % par rapport à la section transversale Qₙ pour une surpression allant jusqu'à 15 mbar agissant sur le côté extérieur du film lorsqu'aucun air vocal ne circule à travers le conduit d'air vocal.

2. Dispositif selon la revendication 1, **caractérisé en ce que** le conduit d'air vocal est formé au moins par portions par une couche (11) du film qui est disposée du côté extérieur de la paroi du tube de canule.

3. Dispositif selon la revendication 1, **caractérisé en ce que** le conduit d'air vocal est formé au moins par portions par un tuyau (12) qui comprend le film et qui est disposé du côté extérieur de la paroi du tube de canule.

4. Dispositif selon la revendication 1, **caractérisé en ce que** le conduit d'air vocal dans la portion de stomie est formé au moins par portions par un manchon gonflable (13) qui comprend le film, qui s'étend annulairement autour du tube de canule et qui comporte au moins une sortie d'air (10) en direction de la portion trachéale.

5. Dispositif selon la revendication 4, **caractérisé en ce que** le manchon gonflable a un diamètre extérieur au moins 20 % supérieur au diamètre extérieur du tube de canule au niveau du manchon pour un débit d'air de 5 litres par minute.

6. Dispositif selon l'une des revendications 1 à 3, **caractérisé en ce que** le conduit d'air vocal est formé aussi bien dans la portion de stomie que par portions dans la portion trachéale par une couche ou par un tuyau comprenant le film, un manchon gonflable (14), qui s'étend annulairement autour du tube de canule et sur la couche ou le tuyau du conduit d'air vocal, étant prévu dans la portion de stomie du côté extérieur de la paroi du tube de canule, la couche ou le tuyau dans la zone qui s'étend à travers le manchon gonflable comportant une sortie d'air supplémentaire (15) dans la lumière du manchon.

7. Dispositif selon l'une des revendications 1 à 6, **caractérisé en ce que**, pour un débit volumique d'air vocal de 5 litres par minute, le diamètre hydraulique du conduit d'air vocal est d'au moins 2,5 mm à une pression ambiante de 1000 mbar.

8. Dispositif selon l'une des revendications 1 à 7, **caractérisé en ce que** le film du conduit d'air vocal est indéchirable au moins jusqu'à une pression d'air vocal de 200 mbar.

9. Dispositif selon l'une des revendications 1 à 8, **caractérisé en ce que** le film du conduit d'air vocal a une épaisseur de paroi dans la gamme allant de 5 à 300 µm .

10. Dispositif selon l'une des revendications 1 à 9, **caractérisé en ce que** le film du conduit d'air vocal au moins dans la zone de stomie est en matière synthétique polymère ayant un Shore A dans la gamme allant de 5 à 90.

11. Dispositif selon l'une des revendications 1 à 10, **caractérisé en ce qu'**il comporte en outre un conduit d'aspiration séparé (16) destiné à aspirer les sécrétions sous-glottiques.

12. Dispositif selon la revendication 11, **caractérisé en ce que** le conduit d'aspiration de sécrétions sous-glottiques est fixé au côté extérieur de la paroi du tube de canule, le film du conduit d'air vocal s'étendant sur le côté supérieur du conduit d'aspiration ou latéralement à côté du conduit d'aspiration ou couvrant au moins par portions dans la direction longitudinale le conduit d'aspiration.

13. Dispositif selon l'une des revendications 1 à 12, **caractérisé en ce que** le conduit d'air vocal comporte un connecteur (17) destiné à de l'air comprimé, de préférence un connecteur bout de doigt, à son extrémité proximale.

14. Dispositif selon l'une des revendications 1 à 13, **caractérisé en ce que** le conduit d'air vocal dans la zone de la portion extracorporelle comprend un tuyau en matière synthétique flexible qui a une dureté et/ou une épaisseur de paroi supérieure à celle(s) du film qu'un conduite d'air vocal comprend au moins par portions dans la portion de stomie et/ou la portion trachéale.

15. Dispositif selon l'une des revendications 1 à 14, **caractérisé en ce que** la conduite d'air vocal comporte une valve (18) à la sortie d'air à son extrémité distale.

16. Dispositif selon l'une des revendications 1 à 15, **caractérisé en ce que** la section transversale de la lumière de la portion n est réduite d'un maximum de 10 % par rapport à la section transversale Qₙ à une surpression allant jusqu'à 15 mbar agissant sur le côté extérieur du film, lorsque l'air vocal circule à travers la conduite d'air vocal avec un débit volumique d'air vocal de 5 litres par minute.
